# EUROPEAN PATENT APPLICATION

(11) **EP 1 136 558 A1**
(43) Date of publication of application: **26.09.2001**
(21) Application number: 00870051.0
(22) Date of filing: 22.03.2000
(51) Int. Cl.: C12N 15/31, C12N 15/82, C07K 14/195, A01H 5/00

(54) **Genetically modified plants and plant cells comprising heterologous heavy metal transport and complexation proteins**

(71) Applicant: "VLAAMSE INSTELLING VOOR TECHNOLOGISCH ONDERZOEK", afgekort "V.I.T.O.", 2400 Mol (BE); LIMBURGS UNIVERSITAIR CENTRUM, 3590 Diepenbeek (BE); VRIJE UNIVERSITEIT BRUSSEL, 1050 Brussel (BE); Sirpa Orvokki Kärenlampi, 70400 Kuopio (FI); Arja Irmeli Tervahauta, 74700 Kiuruvesi (FI)
(72) Inventor: Borremans, Brigitte, 3360 Bierbeek (BE); Bousmans, Nathalie, 1640 Sint-Genesius-Rode (BE); Jacobs, Michel, 1190 Brussels (BE); Kärenlampi, Sirpa Orvokki, 70400 Kuopio (FI); Tervahauta, Arja Irmeli, 74700 Kiuruvesi (FI); Mergeay, Maximilien, 2470 Retie (BE); Van Der Lelie, Daniel, 2400 Mol (BE); Vangronsveld, Jaco, 3590 Diepenbeek (BE)
(74) Representative: Van Malderen, Joelle

(57) **Abstract**

The present invention relates to genetically modified plants and plant cells, comprising nucleotide sequences encoding heterologous heavy metal transport protein.

## Description

### Field of the invention

The present invention is in the field of genetically modified plants and plants cells having improved heavy metal tolerance and accumulation due to increased plant growth and biomass production based upon the expression of exo-cytoplasmic heavy metal resistance system (efflux and complexation).

More particularly, the present invention is related to genetically modified plants and plant cells, comprising nucleotide sequences encoding heterologous heavy metal transport proteins and exocytoplasmic metal binding proteins of various origins.

### Background of the invention and state of the art

Heterologous nucleic acid sequences, coding for heavy metal resistance, were functionally expressed in plants, to improve their tolerance against these toxic elements. The heterologous heavy metal resistance genes, in casu represent either heavy metal efflux systems or functions involved in heavy metal sequestration.

Until present, only cytoplasmic functions that provide increased heavy metal resistance were expressed in plants :

### 1. Expression of heterologous metallothionein and phytochelatines in plants

Metallothioneins and phytochelatines, which are rich in cystein sulfhydryl residues that bind and sequester heavy metal ions in very stable complexes (Karin, 1985), are found in eukaryotic organisms, but recently also in *Synechococcus.* Various MT genes - mouse MTI, human MTIA (alpha domain), human MTII, Chinese hamster MTII, yeast CUP1, pea PsMTA - have been transferred to tobacco, cauliflower or *Arabidopsis thaliana* (Lefebre et al., 1987; Maiti et al., 1988, 1989, 1991; Misra and Gedamu, 1989; Evans et al., 1992; Yeargan et al., 1992; Brandle et al., 1993; Pan et al., 1993; Elmayan and Tepfer, 1994; Hattori et al., 1994; Pan et al., 1994a, b; Hasegawa et al., 1997). As a result, varying degrees of enhanced Cd tolerance have been achieved, being maximally 20-fold compared with the control. Metal uptake levels were not dramatically changed; in some cases there were no differences, in others maximally 70% less or 60% more Cd was taken up by the shoots or leaves. Only one study has been reported on a transgenic plant generated with MT of plant origin. When pea (*Pisum sativum)* MT-like gene PsMTA was expressed in *Arabidopsis thaliana*, more Cu (several-fold in some plants) accumulated in transformed than in control plants (Evans et al., 1992).

### 2. Heterologous expression of heavy metal reduction

The only example known is the *mer* operon of *Tn21* of *Shigella* flexneri, whose expression in plants results in the reduction mercury (Hg²⁺) in its metallic form (Hg⁰). This metallic mercury is volatilized out of the cell (Rugh *et al*. 1996).

### Aims of the invention

The present invention aims to provide a new way in obtaining plants and plant cells with improved heavy metal tolerance characteristics, and possibly heavy metal accumulation.

Another aim of the present invention is to provide such plants and plant cells which allow increased heavy metal resistance for revegetation and phytostabilisation of heavy metal contaminated sites.

A further aim of the present invention is to provide plants and plant cells, characterised by increased heavy metal accumulation combined with increased heavy metal tolerance which allow phytoextraction of heavy metals (inclusive rhizofiltration).

A last aim of the present invention is to provide a method which results in the possibility to improve important agriculture crop species with high biomass production in their heavy metal tolerance and accumulation.

### Summary of the invention

The present invention is related to genetically modified plants and plant cells, comprising nucleotide sequences encoding one or more heterologous heavy metal transport and/or sequestration proteins of various prokaryotic or eukaryotic origins.

Said transporters are preferably membrane proteins, which result in reduced toxicity due to the efflux of heavy metals from the cells, being preferably selected from the group consisting of P-type ATPases, 3 component efflux pumps, ABC transporters and CDF proteins (Cation Diffusion Facilitator proteins).

The family of the P-type ATPases is preferred, because of their advantage that for functional resistance only one protein is required.

Said proteins are found in both prokaryotic and eukaryotic organisms including plants.

Another advantage of said transporters is found as resistance mechanisms against many toxic trace elements of environmental concern, such as copper, cadmium, lead, zinc and silver.

Unexpectedly, it was not necessary to make structural changes in the coding sequence of said proteins, like it is necessary for the merA gene in order to obtain functional expression in plants (Rugh et al., 1996).

Preferably, the gene incorporated in the plants or plant cells is a gene encoding a bacterial P-type ATPase, preferably the cadmium ATPase, such as the *cadA* gene.

According to a second embodiment of the present invention, the system is based upon a prokaryotic heavy metal sequestration system, such as the *pcoA* family protein (more preferably the *pcoA* gene).

The various nucleotide sequences encoding heterologous heavy metal transport proteins can be deleted partially from non-specific nucleotide sequences which are not involved in efficient heavy metal transport or accumulation.

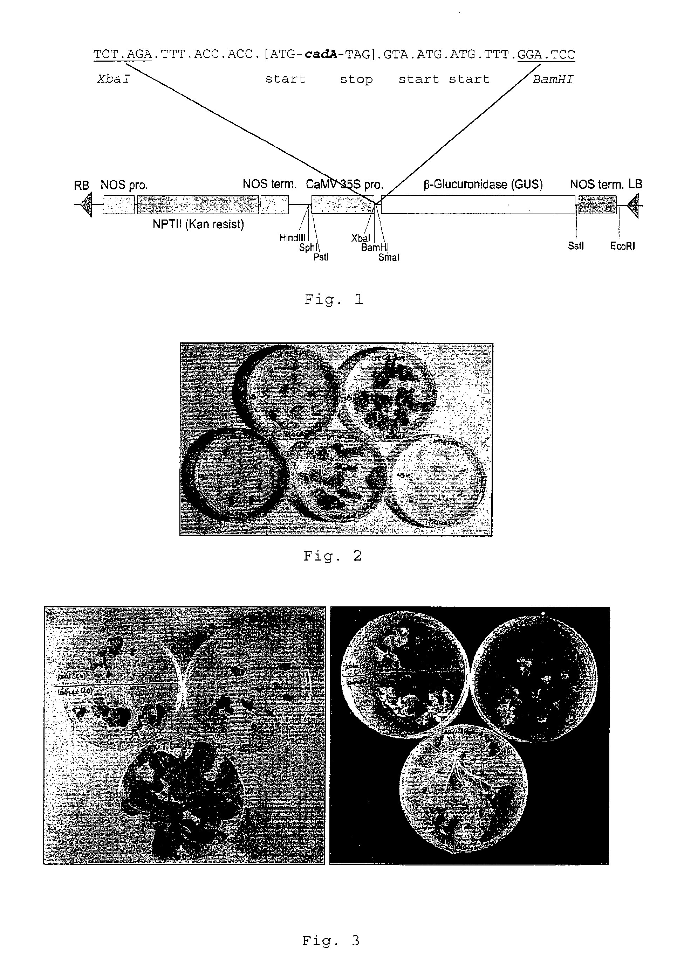

Said genetic sequences could be incorporated in a vector for the transfection of said plants or plant cells, such as the pBI121 vector, as described in the figure 1, said vector being advantageously an *E. coli/Agrobacterium*/plant shuttle vector, said vector comprising preferably a CaMV 35S promoter (a strong promoter constitutively expressed in plants).

Preferably, the system was introduced in the plants, such system allowing the transformation of plants with the *Agrobacterium tumefaciens* technology.

### Short description of the drawings

Fig. 1 is a schematic representation of the cloning of *cadA* in pBI121.

Fig. 2 is a leaf disk-test with Nt WT SR1 (wild type), Nt PBI14 (pBI121) and Nt Cd 309 (pBI121-*cadA*) on 350 *µ*M Cd and control medium without Cd.

Fig. 3 represents the regeneration and growth of Nt WT SR1 (wild type), Nt PBI14 (pBI121) and Nt Cu122 (pBI121-pcoA) on 100 *µ*M Cu, the plant growth being shown from above (left) and top (right).

### Detailed description of the invention

### Heterologous expression of cadA

The heavy metal efflux system was CadA, a member of the P-type heavy metal efflux ATPase family of proteins found both in prokaryotic and eukaryotic organisms. P-type ATPases are all cation pumps, either for uptake, for efflux or for cation exchange. These enzymes have a conserved aspartate residue that is transiently phosphorylated from ATP during the transport cycle, hence the name 'P-type' ATPase (Silver *et al*., 1993).

The *cadA* gene from *Staphylococcus aureus* was amplified by PCR and cloned in the pBI121 vector.

During PCR, appropriate plant specific translation signals were added as well as XbaI and BamHI restriction sites, allowing cloning of the insert in the correct orientation.

The *cadA* fragment was cloned in the *Escherichia coli/Agrobacterium/plant* shuttle vector pBI121. In this vector, *cadA* expression is derived from the CaMV35S promotor, a strong promoter constitutively expressed in plants. The system was introduced in the plant *Nicotiana tabacum* cv. Petit Havana line SR1 via an *Agrobacterium tumefaciens* transformation (Horsch *et al*., 1985). The selection marker used was kanamycine.

Kanamycine resistant transformants were obtained after transformation. All the kanamycine resistant transformants tested showed an increased resistance to cadmium (tested by a leaf disk assay) compared to the wild type and transformant with the pBI121 vector without gene (fig. 1). This proves that the CadA P-type ATPase can be functionally expressed in plants, resulting in an increased resistance of the plant to the trace element (in casu cadmium).

It can be expected that for other members of the P-type ATPase family, which form a family of closely related proteins (both structural and functional) the same positive effect on resistance to specific trace elements will be found. Until present, P-type ATPases from both prokaryotic and eukaryotic have been identified that were found to interact with Zn, Cd, Pb, Cu and Ag (see table 1). It can not be excluded that P-type ATPases, encoding resistance to other trace elements including radioisotopes, will be identified.

**Table 1:**

| *different representatives of the family of P-type ATPases, from prokaryotic and eukariotic origin, which encode resistance against trace elements such as Zn, Cd, Pb, Cu and Ag.* | | | |
|---|---|---|---|
| **Gene** | **Sequence ID** | **Metals** | **Reference** |
| CadA | P20021 | Cd, Zn and Pb | Nucifora *et al.* 1989 Rensing *et al.* 1998 |
| ZntA | P37617 | Zn and Pb | Rensing *et al.* 1997 Rensing *et al.* 1998 |
| CopF | Non available | Cu | van der Lelie and Borremans unpublished |
| PbrA | Not available | Pb | Borremans et al, 2000 |
| SilP | AF067954, nucleotide sequence *sil* operon | Ag | Gupta *et al,* 1999 |
| Menkes' disease | Q04656 | Cu | Vulpe *et al.* 1993 |
| Wilsons' disease | U08344 | Cu | Pethrukin et al. 1993 |

### Heterologous expression of pcoA

The other heavy metal resistance system is involved in exo-cytoplasmic heavy metal sequestration. The tested gene here was *pcoA* from *Escherichia coli* (Brown *et al.,* 1995), which was also cloned in pBI121 and introduced in *Nicotiana tabacum* through an *Agrobacterium tumefaciens* transformation in a way similar as described for *cadA*. Kanamycine resistant transformants were obtained after transformation. All the kanamycine resistant transformants tested showed an increased resistance to copper (tested by a leaf disk assay) compared to the wild type and transformant with the pBI121 vector without gene (Fig. 3).

The *pcoA* protein has many closely related members, found to be involved in resistance against Cu. In addition, other proteins of these copper resistance determinants have also been shown to be involved in Cu sequestration, such as PcoC/CopC and CopE. These proteins, although different in structure, are also active in the bacterial periplasm and possess similar heavy metal binding sites as *pcoA.* In addition, a CopE like protein, referred to as SilE, was identified in the *Salmonella sil* operon encoding for Ag-resistance. The potential genes whose heterologous expression can result in improved resistance, are summarised in table 2.

| **Genes** | **Sequence ID** | **Metals** | **References** |
|---|---|---|---|
| *cop* operon (*copA*, C) e.g. of *Pseudomonas syringae* | M19930 | Cu | Mellano and Cooksey (1988) |
| *pco* operon (*pcoA*, C) of e.g. *E. coli* | G619126 | Cu | Brown *et al.,* 1995 |
| *PcoE* | X83541 | Cu | Brown *et al.,* 1995 |
| *sil* operon of *Salmonella* | AF067954, nucleotide sequence *sil* operon | Ag | Gupta *et al.,* 1999 |

### REFERENCES

- Silver S. et al. (1993). Human Menkes X-chromosome disease and the Staphylococcal cadmium-resistance ATPase: a remarkable similarity in protein sequence; Molecular Microbiology 10(1): 7 - 12.
- Horsch R.B. et al. (1985). A simple and general method for transferring genes into plants; Science 227: 1229 - 1231.
- Karin M (1985). Metallothioneins: Proteins in search of function; Cell 41, 9 - 10.
- Brown N.L. et al. (1995). Molecular genetics and transport analysis of the copper-resistance determinant (pco) from Escherichia coli plasmid pRJ1004; Molecular Microbiology 17(6): 1153 - 1166.
- Mellano, M.A. et al. (1988). Nucleotide sequence and organization of copper resistance genes from *Pseudomonas syringae* pv. *Tomato.* J. Bacteriol. 170: 2879-2883
- Vulpe C.D., et al. (1993). Isolation of a candidate gene for Menkes disease and evidence that it encodes a copper-transporting ATPase; Nature genet. 3: 7 - 13.
- Petrukhin, K. et al. (1993). Mapping, cloning and genetic characterization of the region containing the Wilson disease gene; Nature Genet. 5 (4): 338-343.
- Rugh C.L. et al. (1996). Mercuric reduction and resistance in transgenic *Arabidopsis thaliana* plants expressing a modified bacterial merA gene; Proc.Natl.Acad.Sci.USA 93: 3182 - 3187.
- Lefebvre, D. D. et al, 1987. Mammalian metallothionein functions in plants. Bio/Technology 5, 1053-1056.
- Maiti, I. B. et al, 1988. Seed-transmissable expression of mammalian metallothionein in transgenic tobacco. Biochemical and Biophysical Research Communications 150, 640-647.
- Maiti, I. B. et al, 1989. Inheritance and expression of the mouse metallothionein gene in tobacco. Plant Physiology 91, 1020-1024.
- Maiti, I. B. et al. 1991. Light-inducible and tissue-specific expression of a chimeric mouse metallothionein cDNA gene in tobacco. Plant Science 76, 99-107.
- Misra, S. et al. 1989. Heavy metal tolerant transgenic *Brassica napus* L. and *Nicotiana tabacum* L. plants. Theoretical and Applied Genetics 78, 161-168.
- Evans, K. M. et al 1992. Expression of the pea metallothionein-like gene PsMTA in *Escherichia coli* and *Arabidopsis thaliana* and analysis of trace metal ion accumulation: implications for PsMTA function. Plant Molecular Biology 20, 1019-1028.
- Yeargan, R. et al 1992. Tissue partitioning of cadmium in transgenic tobacco seedlings and field grown plants expressing the mouse metallothionein I gene. Transgenic Research 1, 261-267.
- Nucifora G. et al. (1989) Cadmium resistance from *Staphylococcus aureus* plasmid pI258 cadA gene results from a cadmium-efflux ATPase. Proc. Natl. Acad. Sci. U.S.A. 86 (10): 3544-3548
- Rensing C., et al. (1998). Pb²⁺ (II)-translocating P-type ATPases. J. Biol. Chem. 273: 32614-32617.
- Rensing C., et al. (1997). The *zntA* gene of *Escherichia coli* encodes a Zn(II)-translocating P-type ATPase. Proc. Natl. Acad. Sci. U.S.A. 94 (26): 14326-14331
- Gupta, A., et al. 1999. Molecular basis for resistance to silver cations in *Salmonella.* Nature Medicine 5: 183-188.

### Annex to the application documents - subsequently filed sequences listing

SEQUENCE LISTING

<110> Vlaamse Instelling voor Technologisch Onderzoek (V

<120> GENETICALLY MODIFED PLANTS AND PLANT CELLS COMPRISING
      HETEROLOGOUS HEAVY METAL TRANSPORT AND COMPLEXATION
      PROTEINS

<130> P.VITO.25/EP

<140> 00870051.0
<141> 2000-03-22

<160> 2

<170> PatentIn Ver. 2.1

<210> 1
<211> 2217
<212> DNA
<213> Artificial Sequence

<220>
<221> CDS
<222> (16)..(2196)
<223> cadA

<220>
<221> misc_feature
<222> (1)..(6)
<223> Xba I Restriction site

<220>
<221> misc_feature
<222> (2212)..(2217)
<223> BamH I Restriction site

<220>
<223> Description of Artificial Sequence: Staphylococcus
      aureus cadA gene with BamHI and XbaI restriction
      sites and plant specific translation signals.

<220>
<221> misc_signal
<222> (2206)..(2208)
<223> start signal

<220>

<221> misc_signal
<222> (2203)..(2205)
<223> start signal

<400> 1
tctagattta ccacc atg tct gaa caa aag gtt aaa cta atg gaa gaa gaa 51
Met Ser Glu Gln Lys Val Lys Leu Met Glu Glu Glu
1               5                   10

atg aac gtc tat cgg gtc caa gga ttt aca tgt gca aat tgt gca gga 99
Met Asn Val Tyr Arg Val Gln Gly Phe Thr Cys Ala Asn Cys Ala Gly
        15                  20                  25

aag ttt gag aaa aat gtt aaa aag att cca ggc gtt cag gac gca aaa 147
Lys Phe Glu Lys Asn Val Lys Lys Ile Pro Gly Val Gln Asp Ala Lys
    30                  35                  40

gta aac ttt ggc gct tct aaa att gat gta tat gga aat gca tcg gtt 195
Val Asn Phe Gly Ala Ser Lys Ile Asp Val Tyr Gly Asn Ala Ser Val
45                  50                  55                  60

gaa gaa ctt gaa aaa gca ggt gct ttt gag aat cta aaa gta tct cct 243
Glu Glu Leu Glu Lys Ala Gly Ala Phe Glu Asn Leu Lys Val Ser Pro
                65                  70                  75

gaa aaa cta gcg aat caa acg ata caa agg gtt aaa gat gac act aag 291
Glu Lys Leu Ala Asn Gln Thr Ile Gln Arg Val Lys Asp Asp Thr Lys
            80                  85                  90

gct cat aaa gaa gag aaa aca cca ttt tat aaa aaa cat agt aca ttg 339
Ala His Lys Glu Glu Lys Thr Pro Phe Tyr Lys Lys His Ser Thr Leu
        95                  100                 105

ctg ttt gcc aca cta cta att gct ttt ggt tac ctt tct cac ttt gta 387
Leu Phe Ala Thr Leu Leu Ile Ala Phe Gly Tyr Leu Ser His Phe Val
    110                 115                 120

aat gga gaa gat aac ctc gta act tcc atg tta ttt gta ggt tct att 435
Asn Gly Glu Asp Asn Leu Val Thr Ser Met Leu Phe Val Gly Ser Ile
125                 130                 135                 140

gta att ggc gga tat tca tta ttt aaa gtc ggt ttt caa aat ttg ata 483
Val Ile Gly Gly Tyr Ser Leu Phe Lys Val Gly Phe Gln Asn Leu Ile
                145                 150                 155

cgc ttt gat ttc gac atg aaa acc ctg atg acc gtt gcc gtt att gga 531
Arg Phe Asp Phe Asp Met Lys Thr Leu Met Thr Val Ala Val Ile Gly
            160                 165                 170

gct acc att att ggt aaa tgg gca gag gca tct att gtt gtt att ctc 579
Ala Thr Ile Ile Gly Lys Trp Ala Glu Ala Ser Ile Val Val Ile Leu
        175                 180                 185

ttt gca atc agt gaa gca ctt gaa cgc ttc tct atg gac aga tca aga 627
Phe Ala Ile Ser Glu Ala Leu Glu Arg Phe Ser Met Asp Arg Ser Arg
    190                 195                 200

caa tcc ata cgt tca ttg atg gat atc gcc cca aaa gaa gca cta gtt 675
Gln Ser Ile Arg Ser Leu Met Asp Ile Ala Pro Lys Glu Ala Leu Val
205                 210                 215                 220

aga cga aat ggt cag gaa ata ata atc cat gtg gac gat atc gct gtg 723
Arg Arg Asn Gly Gln Glu Ile Ile Ile His Val Asp Asp Ile Ala Val
                225                 230                 235

ggt gat atc atg att gtc aaa cca ggg gag aaa att gcc atg gat gga 771
Gly Asp Ile Met Ile Val Lys Pro Gly Glu Lys Ile Ala Met Asp Gly
            240                 245                 250

atc att gtg aat ggc ttg tcg gct gtc aat cag gca gct ata aca gga 819
Ile Ile Val Asn Gly Leu Ser Ala Val Asn Gln Ala Ala Ile Thr Gly
        255                 260                 265

gaa tct gtt ccc gtc tcc aaa gcg gta gat gac gaa gta ttt gca ggt 867
Glu Ser Val Pro Val Ser Lys Ala Val Asp Asp Glu Val Phe Ala Gly
    270                 275                 280

acg ctt aac gaa gag gga cta att gaa gta aaa atc acc aaa tac gta 915
Thr Leu Asn Glu Glu Gly Leu Ile Glu Val Lys Ile Thr Lys Tyr Val
285                 290                 295                 300

gaa gat aca acc att acc aag att att cat ctt gtt gaa gaa gca caa 963
Glu Asp Thr Thr Ile Thr Lys Ile Ile His Leu Val Glu Glu Ala Gln
                305                 310                 315

ggg gag cgt gct cca gcc caa gca ttc gtt gat aaa ttt gcg aaa tac 1011
Gly Glu Arg Ala Pro Ala Gln Ala Phe Val Asp Lys Phe Ala Lys Tyr
            320                 325                 330

tac act ccg atc att atg gtt att gca gcc ttg gtt gca gtc gtt cca 1059
Tyr Thr Pro Ile Ile Met Val Ile Ala Ala Leu Val Ala Val Val Pro
        335                 340                 345

ccc cta ttc ttt ggt ggc agt tgg gat aca tgg gtt tat caa gga tta 1107
Pro Leu Phe Phe Gly Gly Ser Trp Asp Thr Trp Val Tyr Gln Gly Leu
    350                 355                 360

gca gtt ctt gta gtt gga tgt cct tgt gca tta gtt att tct act cca    1155
Ala Val Leu Val Val Gly Cys Pro Cys Ala Leu Val Ile Ser Thr Pro
365                 370                 375                 380

atc tcg att gtc tcg gca att gga aat gca gcg aaa aaa ggt gtg ttg    1203
Ile Ser Ile Val Ser Ala Ile Gly Asn Ala Ala Lys Lys Gly Val Leu
                385                 390                 395

gtt aaa ggt ggt gtc tat ctc gag aaa tta gga gcc att aag aca gtc    1251
Val Lys Gly Gly Val Tyr Leu Glu Lys Leu Gly Ala Ile Lys Thr Val
            400                 405                 410

gca ttt gat aaa aca gga aca ctg aca aaa ggt gta cca gtg gta aca    1299
Ala Phe Asp Lys Thr Gly Thr Leu Thr Lys Gly Val Pro Val Val Thr
        415                 420                 425

gat ttt gaa gta tta aat gac caa gtg gaa gaa aaa gag cta ttc tct    1347
Asp Phe Glu Val Leu Asn Asp Gln Val Glu Glu Lys Glu Leu Phe Ser
    430                 435                 440

atc att aca gct tta gaa tat cgt tca caa cat cca ctt gct tca gca    1395
Ile Ile Thr Ala Leu Glu Tyr Arg Ser Gln His Pro Leu Ala Ser Ala
445                 450                 455                 460

ata atg aaa aag gca gag caa gat aat atc cct tat tct aat gta caa    1443
Ile Met Lys Lys Ala Glu Gln Asp Asn Ile Pro Tyr Ser Asn Val Gln
                465                 470                 475

gtg gaa gaa ttc act tcg att act ggg cga ggt ata aaa ggg att gta    1491
Val Glu Glu Phe Thr Ser Ile Thr Gly Arg Gly Ile Lys Gly Ile Val
            480                 485                 490

aac gga act act tac tat att gga agc cca aaa ctt ttc aag gaa tta    1539
Asn Gly Thr Thr Tyr Tyr Ile Gly Ser Pro Lys Leu Phe Lys Glu Leu
        495                 500                 505

aat gtt tcc gat ttt agc ctt ggg ttt gaa aac aat gtg aaa atc cta    1587
Asn Val Ser Asp Phe Ser Leu Gly Phe Glu Asn Asn Val Lys Ile Leu
    510                 515                 520

caa aac caa gga aaa aca gcc atg att att gga acg gaa aaa aca att    1635
Gln Asn Gln Gly Lys Thr Ala Met Ile Ile Gly Thr Glu Lys Thr Ile
525                 530                 535                 540

ctc ggc gta att gcc gtt gca gat gag gtt cgt gaa aca agt aaa aat    1683
Leu Gly Val Ile Ala Val Ala Asp Glu Val Arg Glu Thr Ser Lys Asn
                545                 550                 555

gtg att caa aaa ctt cat cag tta ggt atc aag caa aca att atg ctg    1731
Val Ile Gln Lys Leu His Gln Leu Gly Ile Lys Gln Thr Ile Met Leu
            560                 565                 570

aca ggt gat aat caa ggt act gca aat gca atc ggt aca cat gta ggc    1779
Thr Gly Asp Asn Gln Gly Thr Ala Asn Ala Ile Gly Thr His Val Gly
        575                 580                 585

gtt tct gat att cag tct gaa ttg atg cca cag gat aaa tta gat tat    1827
Val Ser Asp Ile Gln Ser Glu Leu Met Pro Gln Asp Lys Leu Asp Tyr
    590                 595                 600

att aaa aaa atg caa tcg gag tat gat aat gta gct atg att ggc gat    1875
Ile Lys Lys Met Gln Ser Glu Tyr Asp Asn Val Ala Met Ile Gly Asp
605                 610                 615                 620

ggc gtt aat gat gct cca gca ctt gct gca tct act gtt gga att gca    1923
Gly Val Asn Asp Ala Pro Ala Leu Ala Ala Ser Thr Val Gly Ile Ala
                625                 630                 635

atg ggc ggt gct gga acg gat act gca att gaa aca gct gat att gca    1971
Met Gly Gly Ala Gly Thr Asp Thr Ala Ile Glu Thr Ala Asp Ile Ala
            640                 645                 650

tta atg gga gat gat tta agt aag ctt cca ttt gca gta aga ctc agt    2019
Leu Met Gly Asp Asp Leu Ser Lys Leu Pro Phe Ala Val Arg Leu Ser
        655                 660                 665

cga aaa act tta aat atc att aaa gct aac atc act ttt gct atc gga    2067
Arg Lys Thr Leu Asn Ile Ile Lys Ala Asn Ile Thr Phe Ala Ile Gly
    670                 675                 680

att aaa ata att gcc tta cta tta gtt atc ccg gga tgg tta acc ctt    2115
Ile Lys Ile Ile Ala Leu Leu Leu Val Ile Pro Gly Trp Leu Thr Leu
685                 690                 695                 700

tgg ata gcg att ctt tcc gat atg gga gct act att ttg gta gca tta    2163
Trp Ile Ala Ile Leu Ser Asp Met Gly Ala Thr Ile Leu Val Ala Leu
                705                 710                 715

aat agt tta cga ctg atg aga gtg aag gat aaa taggtaatga tgtttggatc 2216
Asn Ser Leu Arg Leu Met Arg Val Lys Asp Lys
            720                 725

c                                                                   2217

<210> 2
<211> 727
<212> PRT
<213> Artificial Sequence
<223> Description of Artificial Sequence: Staphylococcus
      aureus cadA gene with BamHI and XbaI restriction
      sites and plant specific translation signals.

<400> 2
Met Ser Glu Gln Lys Val Lys Leu Met Glu Glu Glu Met Asn Val Tyr
 1               5                  10                  15

Arg Val Gln Gly Phe Thr Cys Ala Asn Cys Ala Gly Lys Phe Glu Lys
            20                  25                  30

Asn Val Lys Lys Ile Pro Gly Val Gln Asp Ala Lys Val Asn Phe Gly
        35                  40                  45

Ala Ser Lys Ile Asp Val Tyr Gly Asn Ala Ser Val Glu Glu Leu Glu
    50                  55                  60

Lys Ala Gly Ala Phe Glu Asn Leu Lys Val Ser Pro Glu Lys Leu Ala
65                  70                  75                  80

Asn Gln Thr Ile Gln Arg Val Lys Asp Asp Thr Lys Ala His Lys Glu
                85                  90                  95

Glu Lys Thr Pro Phe Tyr Lys Lys His Ser Thr Leu Leu Phe Ala Thr
            100                 105                 110

Leu Leu Ile Ala Phe Gly Tyr Leu Ser His Phe Val Asn Gly Glu Asp
        115                 120                 125

Asn Leu Val Thr Ser Met Leu Phe Val Gly Ser Ile Val Ile Gly Gly
    130                 135                 140

Tyr Ser Leu Phe Lys Val Gly Phe Gln Asn Leu Ile Arg Phe Asp Phe
145                 150                 155                 160

Asp Met Lys Thr Leu Met Thr Val Ala Val Ile Gly Ala Thr Ile Ile
                165                 170                 175

Gly Lys Trp Ala Glu Ala Ser Ile Val Val Ile Leu Phe Ala Ile Ser
            180                 185                 190

Glu Ala Leu Glu Arg Phe Ser Met Asp Arg Ser Arg Gln Ser Ile Arg
        195                 200                 205

Ser Leu Met Asp Ile Ala Pro Lys Glu Ala Leu Val Arg Arg Asn Gly
    210                 215                 220

Gln Glu Ile Ile Ile His Val Asp Asp Ile Ala Val Gly Asp Ile Met
225                 230                 235                 240

Ile Val Lys Pro Gly Glu Lys Ile Ala Met Asp Gly Ile Ile Val Asn
                245                 250                 . 255

Gly Leu Ser Ala Val Asn Gln Ala Ala Ile Thr Gly Glu Ser Val Pro
            260                 265                 270

Val Ser Lys Ala Val Asp Asp Glu Val Phe Ala Gly Thr Leu Asn Glu
        275                 280                 285

Glu Gly Leu Ile Glu Val Lys Ile Thr Lys Tyr Val Glu Asp Thr Thr
    290                 295                 300

Ile Thr Lys Ile Ile His Leu Val Glu Glu Ala Gln Gly Glu Arg Ala
305                 310                 315                 320

Pro Ala Gln Ala Phe Val Asp Lys Phe Ala Lys Tyr Tyr Thr Pro Ile
                325                 330                 335

Ile Met Val Ile Ala Ala Leu Val Ala Val Val Pro Pro Leu Phe Phe
            340                 345                 350

Gly Gly Ser Trp Asp Thr Trp Val Tyr Gln Gly Leu Ala Val Leu Val
        355                 360                 365

Val Gly Cys Pro Cys Ala Leu Val Ile Ser Thr Pro Ile Ser Ile Val
    370                 375                 380

Ser Ala Ile Gly Asn Ala Ala Lys Lys Gly Val Leu Val Lys Gly Gly
385                 390                 395                 400

Val Tyr Leu Glu Lys Leu Gly Ala Ile Lys Thr Val Ala Phe Asp Lys
                405                 410                 415

Thr Gly Thr Leu Thr Lys Gly Val Pro Val Val Thr Asp Phe Glu Val
            420                 425                 430

Leu Asn Asp Gln Val Glu Glu Lys Glu Leu Phe Ser Ile Ile Thr Ala
        435                 440                 445

Leu Glu Tyr Arg Ser Gln His Pro Leu Ala Ser Ala Ile Met Lys Lys
    450                 455                 460

Ala Glu Gln Asp Asn Ile Pro Tyr Ser Asn Val Gln Val Glu Glu Phe
465                 470                 475                 480

Thr Ser Ile Thr Gly Arg Gly Ile Lys Gly Ile Val Asn Gly Thr Thr
                485                 490                 495

Tyr Tyr Ile Gly Ser Pro Lys Leu Phe Lys Glu Leu Asn Val Ser Asp
            500                 505                 510

Phe Ser Leu Gly Phe Glu Asn Asn Val Lys Ile Leu Gln Asn Gln Gly
        515                 520                 525

Lys Thr Ala Met Ile Ile Gly Thr Glu Lys Thr Ile Leu Gly Val Ile
    530                 535                 540

Ala Val Ala Asp Glu Val Arg Glu Thr Ser Lys Asn Val Ile Gln Lys
545                 550                 555                 560

Leu His Gln Leu Gly Ile Lys Gln Thr Ile Met Leu Thr Gly Asp Asn
                565                 570                 575

Gln Gly Thr Ala Asn Ala Ile Gly Thr His Val Gly Val Ser Asp Ile
            580                 585                 590

Gln Ser Glu Leu Met Pro Gln Asp Lys Leu Asp Tyr Ile Lys Lys Met
        595                 600                 605

Gln Ser Glu Tyr Asp Asn Val Ala Met Ile Gly Asp Gly Val Asn Asp
    610                 615                 620

Ala Pro Ala Leu Ala Ala Ser Thr Val Gly Ile Ala Met Gly Gly Ala
625                 630                 635                 640

Gly Thr Asp Thr Ala Ile Glu Thr Ala Asp Ile Ala Leu Met Gly Asp
                645                 650                 655

Asp Leu Ser Lys Leu Pro Phe Ala Val Arg Leu Ser Arg Lys Thr Leu
            660                 665                 670

Asn Ile Ile Lys Ala Asn Ile Thr Phe Ala Ile Gly Ile Lys Ile Ile
        675                 680                 685

Ala Leu Leu Leu Val Ile Pro Gly Trp Leu Thr Leu Trp Ile Ala Ile
    690                 695                 700

Leu Ser Asp Met Gly Ala Thr Ile Leu Val Ala Leu Asn Ser Leu Arg
705                 710                 715                 720

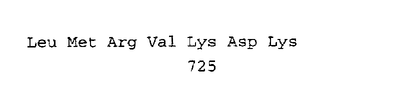

## Claims

1. Genetically modified plants and plant cells, comprising nucleotide sequences encoding one or more heterologous heavy metal transporters or sequestration proteins.

2. Genetically modified plants or plant cells, the nucleotide sequence encoding the heterologous heavy metal transport proteins being genes encoding heavy metal transporters, such as transporters selected from the group consisting of P-type ATPase, 3 components efflux pumps or ABC transporters.

3. Genetically modified plants or plant cells according to the claim 2, **characterised in that** the nucleotide sequence encodes for cadmium ATPase.

4. Genetically modified plants or plant cells according to the claim 2 or 3, wherein the nucleotide sequence is *cad A* or a portion thereof allowing heavy metal transport.

5. Genetically modified plants or plant cells according to the claim 1, **characterised in that** the nucleotide sequence encoding the heavy metal sequestration protein belongs to the copA family.

6. Use of the genetically modified plants or plant cells according to any of the preceding claims for phytoremediation of contaminated sites, especially for the revegetation, phytostabilisation, phytoextraction of soils and/or water contaminated with trace elements.
